# FASCICULE DE BREVET EUROPEEN

(11) **EP 1 133 691 B1**
(45) Date de publication et mention de la délivrance du brevet: **02.04.2003**
(21) Numéro de dépôt: 99923354.7
(22) Date de dépôt: 04.06.1999
(51) Int. Cl.: G01N 33/487, C12M 1/34

(54) **DISPOSITIF DE CULTURE DE CELLULES ORGANIQUES ET D'ETUDE DE LEUR ACTIVITE ELECTROPHYSIOLOGIQUE ET MEMBRANE UTILISEE DANS UN TEL DISPOSITIF**
VORRICHTUNG ZUM ZÜCHTEN VON ORGANISCHEN ZELLEN UND ERFORSCHUNG IHRER ELEKTROPHYSIOLOGISCHEN AKTIVITÄT UND DABEI VERWENDETE MEMBRAN
DEVICE FOR ORGANIC CELL CULTURE AND FOR STUDYING THEIR ELECTROPHYSIOLOGICAL ACTIVITY AND MEMBRANE USED IN SAID DEVICE

(30) Priorité: 08.06.1998 FR 9807596
(43) Date de publication de la demande: 19.09.2001
(73) Titulaire: BIO CELL-INTERFACE S.A., 2300 La Chaux-de-Fonds (CH)
(72) Inventeur: Hänni, Claude, CH-2300 La Chaux-de-Fonds (CH); STOPPINI, Luc, CH-1202 Genève (CH)
(74) Mandataire: Nithardt, Roland
(86) Numéro de dépôt international: CH9900243
(87) Numéro de publication internationale: WO99064559

(56) Documents cités:
- EP-A- 0 689 051
- US-A- 5 759 846
- DATABASE WPI Section Ch, Week 8523 Derwent Publications Ltd., London, GB; Class D16, AN 85-139721 XP002095669 & SU 1 124 022 A (AS USSR BIOEQUIP DE), 15 novembre 1984 (1984-11-15)

## Description

La présente invention concerne un dispositif pour la culture d'amas de cellules organiques et pour l'étude de l'activité électrophysiologique des cellules, dans lequel les cellules sont placées sur au moins une membrane poreuse dont la face inférieure est en contact avec un liquide nutritif, ce dispositif comportant au moins une électrode agencée pour être en contact avec ledit amas de cellules organiques.

La présente invention concerne également une membrane réalisée en une matière synthétique poreuse, ainsi que l'utilisation de cette membrane pour réaliser un modèle de barrière hémato-encéphalique.

Il existe actuellement différents dispositifs permettant de mesurer l'activité électrophysiologique d'amas de cellules organiques.

Un tel dispositif est en particulier décrit dans la demande de brevet français FR-A-2 733 055. Dans ce document, il est décrit un dispositif qui permet de maintenir en vie des explants tissulaire et de recueillir et d'analyser en continu l'activité électrophysiologioque et biochimique du tissu étudié. Ce dispositif est formé de deux demi-cartes formant respectivement la partie supérieure et la partie inférieure de l'interface qui s'assemblent pour former une carte destinée à être insérée dans un boîtier électronique spécialement conçu à cet effet.

Ce dispositif donne des résultats satisfaisants, mais présente toutefois un certain nombre d'inconvénients. En effet, les électrodes sont forcées en direction des cellules jusqu'à ce qu'elles soient en contact avec celles-ci. Ceci blesse un certain nombre de cellules, ce qui diminue leur durée de survie. Après un certain temps, les électrodes et les cellules sont intimement liées. Lorsqu'il est nécessaire de retirer les cellules, par exemple, pour réaliser une analyse au moyen d'un microscope, une partie d'entre elles restent fixées aux électrodes, ce qui a pour effet de détruire la structure de l'amas de cellules et de les rendre ainsi inutilisables.

Dans ce dispositif, les cellules sont nourries par dessous et les électrodes sont posées sur l'amas de cellules. Ces électrodes empêchent donc une visualisation des tissus. Cette visualisation est importante notamment parce qu'elle permet de connaître l'organisation des tissus et de déterminer ainsi les électrodes qui doivent être utilisées. Elle permet également de contrôler la survie de ces tissus.

En outre, le fait de placer les électrodes sur les cellules empêche d'intervenir sur les tissus analysés. De plus, ceci implique que les électrodes doivent avoir une résistance mécanique relativement élevées puisqu'elles sont formées de pistes de cuivre ne reposant pas sur un substrat. En outre, la conception de la carte en deux demi-cartes implique la fabrication et l'assemblage d'un nombre élevé de pièces.

Comme les électrodes sont disposées entre les deux demi-cartes, il faut qu'une de leur extrémité soit amenée dans une zone accessible par un connecteur électrique. Comme chaque carte est à usage unique, le coût d'utilisation dû au nombre de pièces et à la complexité du dispositif est élevé.

D'autres dispositifs comportant un substrat en verre ont également été développés et sont notamment décrits dans la demande de brevet européen publiée sous le No. EP 0 689 051. Dans ces dispositifs, les tissus biologiques doivent être collés pour qu'ils puissent adhérer au substrat. Le substrat n'étant pas poreux, il est nécessaire de placer le dispositif dans un appareillage qui assure un mouvement qui permet au tissu d'être successivement immergé et émergé pour que le tissu puisse respirer. Ce dispositif est lourd et ne permet pas une survie à long terme lorsque l'on arrête le mouvement. En outre, il est difficile de réaliser plusieurs substrats simultanément sur une plaque. La fabrication de tels substrats est particulièrement longue et coûteuse.

La présente invention permet de pallier ces inconvénients en réalisant un dispositif bon marché, comportant un nombre restreint de pièces et permettant d'effectuer une analyse électrophysiologique et/ou microscopique des cellules sans les détruire.

Ces buts sont atteints par un dispositif tel que décrit en préambule et caractérisé en ce que les électrodes sont réalisées sur la membrane poreuse.

Selon un mode de réalisation préféré, le dispositif comporte un réseau d'électrodes.

Chaque électrode comporte avantageusement une zone d'analyse agencée pour pouvoir être disposée en contact avec l'amas de cellules, et une zone de mesure agencée pour pouvoir être mise en contact avec un appareil générant un signal électrique et/ou mesurant un signal électrique.

La membrane poreuse est de préférence maintenue sur un support rigide.

Ce support rigide contient avantageusement une chambre d'alimentation en liquide nutritif, cette chambre communiquant avec un conduit d'entrée et un conduit d'évacuation de liquide nutritif et étant pourvue d'une ouverture communiquant avec ladite membrane poreuse.

Selon une forme de réalisation préférée, la membrane poreuse est surmontée d'une capsule agencée pour maintenir les cellules organiques dans un environnement contrôlé. Cette capsule peut comporter un conduit d'injection de gaz et un conduit d'évacuation de gaz.

Selon un mode de réalisation particulier de l'invention, les zones de mesure des électrodes du réseau d'électrodes sont disposées sur un cercle.

Le réseau d'électrodes comporte avantageusement un moyen d'indexage de sa position.

Selon une forme de réalisation préférée, la membrane poreuse est transparente.

Les buts fixés par l'invention sont également atteints par une membrane telle que définie en préambule et caractérisée en ce qu'elle comporte au moins une électrode déposée sur cette membrane.

Selon une forme de réalisation préférée, la membrane comporte un réseau d'électrodes déposées sur cette membrane.

Selon une forme de réalisation préférée, chaque électrode comporte au moins une zone d'analyse, une zone de mesure et une zone de connexion.

Finalement, les buts de l'invention sont également atteints par un procédé pour réaliser un modèle de barrière hémato-encéphalique à partir d'une membrane telle que définie ci-dessus, ce procédé étant caractérisé en ce qu'il comporte des étapes consistant à traiter la membrane poreuse de façon à permettre à des cellules endothéliales d'y adhérer, à placer des cellules endothéliales sur une face ne comportant pas d'électrode, de la membrane poreuse, à cultiver lesdites cellules endothéliales jusqu'à ce qu'elles forment une couche, et à placer une culture organotypique en tranche sur l'autre face comportant au moins une électrode, de la membrane poreuse.

La présente invention sera mieux comprise en référence à la description d'un mode de réalisation particulier de l'invention et aux dessins annexés dans lesquels :
- la figure 1 est une vue de dessus du dispositif selon la présente invention;
- la figure 2 est une vue agrandie d'une partie du dispositif de la figure 1;
- la figure 3 est une vue en coupe selon la ligne A-A de la figure 1; et
- la figure 4 est une vue en coupe illustrant une utilisation particulière du dispositif de l'invention.

En référence aux figures, le dispositif 10 comporte un support 11 dans l'épaisseur duquel est réalisée une chambre d'alimentation 12 en liquide nutritif. Cette chambre d'alimentation communique avec un conduit d'entrée 13 lié à un système de perfusion (non représenté) de liquide nutritif. Cette chambre communique également avec un conduit d'évacuation 14 dudit liquide nutritif.

Cette chambre comporte également une ouverture 15 dans le haut du support. Une membrane poreuse 16 transparente est placée sur le support 11 de façon à recouvrir l'ouverture 15. Cette membrane poreuse 16 peut être réalisée par exemple en polyéthylène téréphtalate (PET) ou en polycarbonate notamment. Elle comporte un réseau d'électrodes 17 réalisées directement sur la membrane. Chaque électrode 18 du réseau d'électrodes 17 comporte une zone d'analyse 19, une zone de mesure 20 et une zone de connexion 21.

La zone d'analyse 19 est une partie non isolée de l'électrode. Cette zone se trouve au-dessus de l'ouverture 15 de la chambre d'alimentation 12. La zone de mesure 20 est également une partie non isolée de l'électrode. Elle est réalisée, par exemple, sous la forme d'un cercle ayant une surface suffisante pour être facilement mise en contact avec un connecteur.

La zone de connexion 21 est une partie isolée de l'électrode, reliant la zone d'analyse 19 à la zone de mesure 20.

Les électrodes peuvent être réalisées, par exemple, en or ou en platine. Le réseau d'électrodes peut être réalisé selon plusieurs procédés distincts. Dans l'un des procédés, on évapore une couche d'or par exemple, sur la membrane poreuse au moyen d'un procédé connu sous la dénomination de "Plasma vapor deposition" (PVD) ou par évaporation sous vide. On dépose ensuite une couche de matière photorésistante. Cette couche est soumise à une insolation à travers un premier masque photographique reproduisant le réseau d'électrodes. L'ensemble est ensuite révélé. L'or est usiné chimiquement, puis la membrane est rincée avant que la première couche de matière photorésistante ne soit totalement dissolue.

L'isolation de certaines parties du réseau d'électrodes est effectuée de la façon suivante: une couche d'une deuxième matière photorésistante est déposée par trempage sur la membrane. Cette matière subit une cuisson, puis une nouvelle insolation est effectuée à travers un masque photographique reproduisant les zones isolées des électrodes. La membrane subit ensuite un développement, puis un rinçage.

Le réseau d'électrodes peut également être réalisé par évaporation d'or par procédé PVD à travers un premier masque. Le "réseau" d'isolant est ensuite déposé de façon similaire par un procédé PVD à travers un deuxième masque. L'isolation peut par exemple être de l'oxyde de titane.

Chaque électrode peut également comporter une zone non isolée, constituant la zone d'analyse 19, ayant une forme carrée par exemple. Cette zone non isolée est disposée à proximité de l'extrémité de chaque électrode, au-dessus de l'ouverture 15 de la chambre d'alimentation. De l'or ou du platine est ensuite déposé par électrodéposition dans la zone non isolée. Ce procédé est avantageux à différents points de vue. Il permet d'une part d'avoir un bon contact électrique entre les cellules et les électrodes. D'autre part, cela diminue l'impédance des électrodes. Finalement, il est particulièrement facile de modifier la surface active des électrodes, c'est-à-dire la surface de la zone non isolée. Il est également possible de réaliser des électrodes ayant des surfaces actives différentes dans un même réseau d'électrodes.

Dans le mode de réalisation illustré, les zones de mesure des électrodes sont disposées sur un cercle de telle façon qu'elles soient facilement accessibles pour activer les électrodes ou pour mesurer des signaux électriques. Il est clair que d'autres configurations pourraient également être choisies.

Le dispositif 10 comporte en outre une capsule 22 formée d'une paroi latérale et d'un fond partiellement ouvert. Cette capsule est fixée rigidement au-dessus de la membrane poreuse de telle façon que l'ouverture 15 de la chambre d'alimentation communique avec l'ouverture du fond de la capsule 22 par l'intermédiaire de la membrane poreuse 16.

Cette capsule 22 comporte en outre un couvercle 23 qui peut être placé sur la capsule de façon à protéger son contenu du milieu extérieur. Ce couvercle peut être fermé hermétiquement. La capsule peut également comporter un conduit 28 d'injection de gaz et un conduit 29 d'évacuation de gaz. En fonction des mesures à effectuer ou de la nature des cellules ou des produits à tester, un gaz peut être introduit dans ce conduit d'injection de gaz. Il est également possible de générer un flux de gaz dans la chambre en introduisant un gaz dans le conduit d'injection et en l'évacuant par le conduit d'évacuation de gaz.

Le support 11 est rigide et comporte des moyens de maintien 24 et des moyens d'indexage 25 de sa position. Les moyens de maintien 24 peuvent être réalisés sous la forme de deux trous 26 coopérant avec deux tiges d'un boîtier de connexion (non représenté). Ces moyens de maintien permettent d'assurer le maintien en position du dispositif 10 dans ce boîtier de connexion.

Les moyens d'indexage 25 sont, par exemple, formés par une encoche 27 coopérant avec un bossage (non représenté) du boîtier de connexion. Ils permettent d'assurer le positionnement correct du dispositif dans le boîtier de connexion et évitent en particulier que ce dispositif ne soit placé dans une position symétrique par rapport à la position correcte.

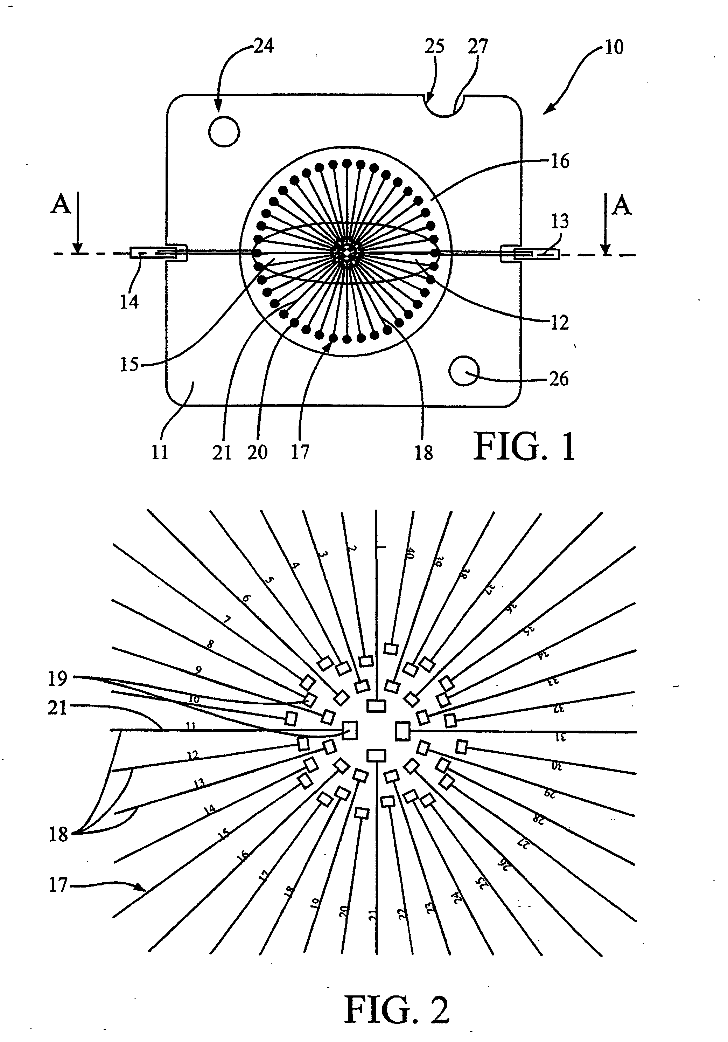

Lors de l'utilisation du dispositif tel qu'illustré par les figures 1 à 3, un amas cellulaire est placé dans la capsule 22. Cet amas peut être placé directement sur la membrane poreuse 16, de façon conventionnelle. Il est toutefois également possible de cultiver des cellules sur une membrane poreuse ayant par exemple une forme circulaire, puis de placer ces cellules avec la membrane circulaire dans la capsule. Ceci est particulièrement intéressant dans le cas où des cellules doivent être cultivées avant de pouvoir procéder à leur analyse. Il est ainsi possible de stimuler et d'enregistrer les réponses électrophysiologiques des cellules à travers la membrane prédécoupée, sans que ce tissu biologique soit en contact direct avec les électrodes.

Un liquide nutritif est amené dans la chambre d'alimentation 12 par le conduit d'entrée 13. Ce liquide entre en contact avec la membrane poreuse et recouvre totalement l'amas cellulaire de liquide nutritif par un film de milieu de culture. Ceci permet une bonne diffusion des gaz dans toute l'épaisseur du tissu, et assure une durée de vie importante des cellules. D'autre part, cela permet d'éviter la nécessité de mettre l'ensemble du dispositif en mouvement, comme dans certains dispositifs de l'art antérieur. Le couvercle 23 de la capsule 22 est généralement maintenu fermé afin d'éviter des pollutions dues au milieu extérieur.

Le film de liquide nutritif a également pour effet de plaquer l'amas cellulaire contre les électrodes, ce qui assure un bon contact électrique entre les électrodes et les cellules, sans qu'il soit nécessaire de coller ces cellules.

Le dispositif est avantageusement placé dans un boîtier de connexion (non représenté) qui relie chacune des zones de mesure des électrodes à une entrée du boîtier de connexion. Ceci permet de transmettre de façon simple, un signal électrique à une ou plusieurs électrodes sélectionnées du réseau d'électrodes, en introduisant ce signal à l'entrée ou aux entrées correspondantes du boîtier de connexion.

De même, ceci permet de mesurer de façon simple, un signal électrique d'une ou plusieurs électrodes du réseau d'électrodes.

Les conduits d'entrée 13 et d'évacuation 14 permettent d'introduire des substances chimiques à tester, tout en maintenant le dispositif dans son environnement dans lequel les mesures électriques sont effectuées.

La membrane poreuse étant transparente, l'amas cellulaire peut être analysé au microscope sans qu'il soit nécessaire de le retirer des électrodes et, par conséquent, sans détruire sa structure. La membrane peut également être retirée du support, ce qui facilite sa manipulation lors de différents tests.

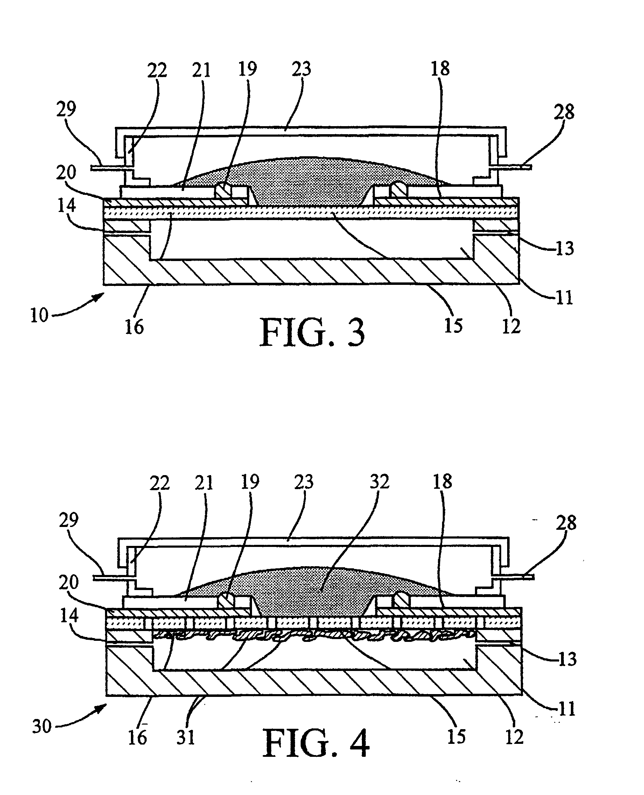

La figure 4 illustre une utilisation particulière du dispositif 30 selon l'invention, dans lequel un modèle de barrière hémato-encéphalique a été réalisé. Cette barrière hémato-encéphalique est formée de cellules endothéliales qui tapissent les capillaires du système nerveux central. Ces cellules ont des propriétés spécifiques par rapport à celles des autres organes. Elles forment une barrière qui empêche le passage de la plupart des molécules hydrosolubles, sauf de celles qui ont un transporteur particulier tel que par exemple le glucose.

Cette barrière joue un rôle important dans la protection des tissus nerveux. Elle empêche parfois le passage de certains médicaments actifs, mais qui ne peuvent pas traverser cette barrière. Pour cette raison, il est important de disposer de modèles de barrières hémato-encéphaliques, pour tester la perméabilité de nouveaux médicaments.

Le modèle de barrière hémato-encéphalique développé avec le dispositif de la présente invention est obtenu par la co-culture de cellules endothéliales 31 et de cultures organotypiques 32 en tranche. Ce modèle de barrière est particulièrement intéressant du fait qu'il permet de connaître en une seule expérience, la perméabilité des molécules et ses effets sur les tissus nerveux.

Comme cela est illustré en détail par la figure 4, la co-culture des cellules endothéliales 31 et de cultures organotypiques 32 est intégrée à la membrane poreuse 16. L'ensemble ainsi formé permet de tester la perméabilité de molécules dans un modèle qui est très proche de la situation in-vivo, mais qui est nettement plus simple et moins coûteux à réaliser.

Dans ce mode de réalisation, la membrane poreuse 16 est tout d'abord traitée de façon à permettre aux cellules endothéliales 31 d'y adhérer. Des cellules endothéliales sont ensuite injectées dans la chambre. Lorsqu'elles forment une couche compacte, une culture organotypique 32 est placée de l'autre côté de la membrane, sur les électrodes 18 formant le réseau d'électrodes. L'ensemble du dispositif est conservé pendant quelques jours dans un incubateur, pendant la durée nécessaire à la formation de la barrière hémato-encéphalique. Le dispositif de l'invention, auquel est ajouté la barrière hémato-encéphalique est utilisé comme décrit précédemment. Les molécules à tester sont injectées dans la chambre par le conduit d'entrée 13.

La perméabilité des molécules neuroactives peut être directement déterminée en analysant les modifications de l'activité électrophysiologique du tissus nerveux, ces modification pouvant apparaître du fait de la présence des molécules à tester dans le tissus. Le réseau d'électrodes permet de stimuler et d'enregistrer simultanément l'activité électrique du tissus nerveux par l'intermédiaire d'un dispositif de traitement adéquat.

Le dispositif selon la présente invention est généralement prévu pour un usage unique. Il est jeté après chaque analyse. Le nombre de pièces qui le compose a été réduit à un minimum. Ceci permet donc de réduire le coût du dispositif.

D'autre part, grâce à l'utilisation d'un substrat souple, il est facile de réaliser plusieurs substrats simultanément, sur une plaque, puis de découper la plaque lorsque les électrodes ont été réalisées. Ceci permet une fabrication industrielle des substrats.

La présente invention n'est pas limitée au mode de réalisation décrit, mais s'étend à toute variante évidente pour un homme du métier. En particulier, la forme de la membrane poreuse 16 pourrait être non circulaire. En utilisant, par exemple, une membrane poreuse carrée, le positionnement des côtés du carré permettrait d'assurer le positionnement des électrodes. Ce positionnement peut être important lorsque le dispositif doit coopérer avec un boîtier de connexion dans lequel la position des connecteurs est fixe.

## Revendications

1. Dispositif pour la culture d'amas de cellules organiques et pour l'étude de l'activité électrophysiologique des cellules, dans lequel les cellules sont placées sur au moins une membrane poreuse dont la face inférieure est en contact avec un liquide nutritif, ce dispositif comportant au moins une électrode agencée pour être en contact avec ledit amas de cellules organiques, **caractérisé en ce que** les électrodes (18) sont réalisées sur la membrane poreuse (16).

2. Dispositif selon la revendication 1, **caractérisé en ce qu'**il comporte un réseau d'électrodes (17).

3. Dispositif selon la revendication 1, **caractérisé en ce que** chaque électrode (18) comporte une zone d'analyse (19) agencée pour pouvoir être disposée en contact avec l'amas de cellules, et une zone de mesure (20) agencée pour pouvoir être mise en contact avec un appareil générant un signal électrique et/ou mesurant un signal électrique.

4. Dispositif selon la revendication 1, **caractérisé en ce que** la membrane poreuse (16) est maintenue sur un support rigide (11).

5. Dispositif selon la revendication 4, **caractérisé en ce que** le support rigide (11) contient une chambre d'alimentation (12) en liquide nutritif, cette chambre communiquant avec un conduit d'entrée (13) et un conduit d'évacuation (14) de liquide nutritif et étant pourvue d'une ouverture (15) communiquant avec ladite membrane poreuse (16).

6. Dispositif selon la revendication 1, **caractérisé en ce que** la membrane poreuse (16) est surmontée d'une capsule (22) agencée pour maintenir les cellules organiques dans un environnement contrôlé.

7. Dispositif selon la revendication 6, **caractérisé en ce que** la capsule (22) comporte un conduit (28) d'injection de gaz et un conduit (29) d'évacuation de gaz.

8. Dispositif selon la revendication 3, **caractérisé en ce que** les zones de mesure (20) des électrodes (18) du réseau d'électrodes (17) sont disposées sur un cercle.

9. Dispositif selon la revendication 1, **caractérisé en ce que** le réseau d'électrodes (17) comporte un moyen d'indexage (25) de sa position.

10. Dispositif selon la revendication 1, **caractérisé en ce que** la membrane poreuse (16) est transparente.

11. Membrane en matière synthétique poreuse comportant au moins une électrode, **caractérisée en ce que** cette électrode (18) est déposée sur la membrane.

12. Membrane selon la revendication 11, **caractérisée en ce qu'**elle comporte un réseau d'électrodes (17) déposées sur cette membrane.

13. Membrane selon la revendication 11 ou 12, **caractérisée en ce que** chaque électrode (18) comporte au moins une zone d'analyse (19), une zone de mesure (20) et une zone de connexion (21).

14. Utilisation d'une membrane selon l'une quelconque des revendications 11 à 13, pour réaliser un modèle de barrière hémato-encéphalique.

15. Procédé pour réaliser un modèle de barrière hémato-encéphalique à partir d'une membrane selon l'une quelconque des revendications 11 à 13, **caractérisé en ce qu'**il comporte des étapes consistant à :
- traiter la membrane poreuse (18) de façon à permettre à des cellules endothéliales (31) d'y adhérer,
- placer des cellules endothéliales (31) sur une face ne comportant pas d'électrode, de la membrane poreuse (18),
- cultiver lesdites cellules endothéliales (31) jusqu'à ce qu'elles forment une couche, et
- placer une culture organotypique en tranche (32) sur l'autre face, comportant au moins une électrode, de la membrane poreuse (18).

## Claims

1. A device for the culture of masses of organic cells and for studying the electrophysiological activity of the cells, in which the cells are placed on at least one porous membrane, the lower surface of which is in contact with a liquid nutrient, this device comprising at least one electrode designed to contact said mass of organic cells, **characterized in that** the electrodes (18) are made on the porous membrane (16).

2. A device according to claim 1, **characterized in that** it comprises a network of electrodes (17).

3. A device according to claim 1, **characterized in that** each electrode (18) comprises an analysis zone (19) designed to be placed in contact with the cell mass and a measurement zone (20) designed to be placed in contact with an apparatus generating an electrical signal and/or measuring an electrical signal.

4. A device according to claim 1, **characterized in that** the porous membrane (16) is held by a rigid support (11).

5. A device according to claim 4, **characterized in that** the rigid support (11) contains a liquid nutrient supply chamber (12), this chamber communicating with a liquid nutrient inlet duct (13) and a liquid nutrient exhaust duct (14) and being provided with an opening (15) which communicates with said porous membrane (16).

6. A device according to claim 1, **characterized in that** a capsule (22) surmounts the porous membrane (16) in order to keep the organic cells in a controlled environment.

7. A device according to claim 6, **characterized in that** the capsule (22) comprises a gas injection duct (28) and a gas exhaust duct (29).

8. A device according to claim 3, **characterized in that** the measurement zones (20) on the electrodes (18) in the electrode network (17) are arranged in a circle.

9. A device according to claim 1, **characterized in that** the electrode network (17) comprises a position indexing means (25).

10. A device according to claim 1, **characterized in that** the porous membrane (16) is transparent.

11. A membrane made of synthetic porous material comprising at least one electrode, **characterized in that** this electrode (18) is deposed on the membrane.

12. A membrane according to claim 11, **characterized in that** it comprises an electrode network (17) deposed on this membrane.

13. A membrane according to claim 11 or 12, **characterized in that** each electrode (18) comprises at least one analysis zone (19), one measurement zone (20) and one connection zone (21).

14. A use of a membrane according to any one of the claims 11 to 13, to make a hemato-encephalic barrier model.

15. A method of making a hemato-encephalic barrier model from a membrane according to any one of the claims 11 to 13, **characterized in that** it comprises the steps of:
- treating the porous membrane (18) so as to permit endothelial cells (31) to adhere to it;
- placing endothelial cells (31) on a surface of the porous membrane (18) that has no electrode;
- cultivating said endothelial cells (31) until they form a layer; and
- placing a slice of an organotypical culture (32) on the other surface of the porous membrane (18) which has at least one electrode.

## Patentansprüche

1. Vorrichtung zur Kultivierung von Ansammlungen organischer Zellen und zum Studium der elektrophysiologischen Aktivität der Zellen, in welcher die Zellen auf mindestens einer porösen Membran positioniert werden, deren Innenseite mit einer Nährfiüssigkeit in Berührung steht, wobei die Vorrichtung mindestens eine Elektrode aufweist, die dafür vorgesehen ist, mit der Ansammlung organischer Zellen in Berührung zu stehen,
**dadurch gekennzeichnet, dass**
die Elektroden (18) auf der porösen Membran (16) angeordnet sind.

2. Vorrichtung nach Anspruch 1,
**dadurch gekennzeichnet, dass**
sie ein Elektrodennetz (17) aufweist.

3. Vorrichtung nach Anspruch 1,
**dadurch gekennzeichnet, dass**
jede Elektrode (18) eine Analysezone (19) umfasst, die dafür vorgesehen ist, mit der Ansammlung von Zellen in Berührung stehend angeordnet zu werden, und eine Messzone (20), die dafür vorgesehen ist, mit einem ein elektrisches Signal erzeugendem und/oder ein elektrisches Signal messendem Gerät in Berührung zu stehen.

4. Vorrichtung nach Anspruch 1,
**dadurch gekennzeichnet, dass**
die poröse Membran (16) auf einem starren Träger (11) gehalten wird.

5. Vorrichtung nach Anspruch 4,
**dadurch gekennzeichnet, dass**
der starre Träger (11) eine Nährflüssigkeitsversorgungskammer (12) enthält, wobei die Kammer mit einer Nährflüssigkeitseinlassleitung (13) und mit einer -auslassleitung (14) in Verbindung steht und eine mit der porösen Membran (16) in Verbindung stehende Öffnung (15) aufweist.

6. Vorrichtung nach Anspruch 1,
**dadurch gekennzeichnet, dass**
die poröse Membran (16) von einer Kapsel (22) überragt wird, die zur Haltung der organischen Zellen in einer kontrollierten Umgebung vorgesehen ist.

7. Vorrichtung nach Anspruch 6,
**dadurch gekennzeichnet, dass**
die Kapsel (22) eine Gaseinspritzleitung (28) und eine Gasauslassleitung (29) aufweist.

8. Vorrichtung nach Anspruch 3,
**dadurch gekennzeichnet, dass**
die Messzonen (20) der Elektroden (18) des Elektrodennetzes (17) kreisförmig angeordnet sind.

9. Vorrichtung nach Anspruch 1,
**dadurch gekennzeichnet, dass**
das Elektrodennetz (17) ein Mittel (25) zur Indexierung seiner Position aufweist.

10. Vorrichtung nach Anspruch 1,
**dadurch gekennzeichnet, dass**
die poröse Membran (16) durchsichtig ist.

11. Membran aus synthetischem porösem Material mit mindestens einer Elektrode,
**dadurch gekennzeichnet, dass**
die Elektrode (18) auf der Membran gelagert ist.

12. Membran nach Anspruch 11,
**dadurch gekennzeichnet, dass**
sie ein auf der Membran gelagertes Elektrodennetz (17) aufweist.

13. Membran nach Anspruch 11 oder 12,
**dadurch gekennzeichnet, dass**
jede Elektrode (18) mindestens eine Analysezone (19), eine Messzone (20) und eine Verbindungszone (21) aufweist.

14. Verwendung einer Membran nach einem der Ansprüche 11 bis 13 zur Ausführung eines Blut-Hirn-Schranke-Modells.

15. Verfahren zur Ausführung eines Blut-Hirn-Schranke-Modells mit einer Membran nach einem der Ansprüche 11 bis 13,
**dadurch gekennzeichnet, dass**
es die sich wie folgt zusammensetzenden Schritte umfasst:
- Behandlung der porösen Membran auf eine Art und Weise, dass ein Anhaften endothelialer Zellen (31) daran ermöglicht wird,
- Positionierung endothelialer Zellen (31) auf einer keine Elektroden aufweisenden Seite der porösen Membran (18),
- Kultivierung der endothelialen Zellen (31), bis sie eine Schicht bilden, und
- Positionierung einer scheibenförmigen organtypischen Kultur (32) auf der mindestens eine Elektrode aufweisenden anderen Seite der porösen Membran (18).
